# EUROPEAN PATENT APPLICATION

(11) **EP 3 401 880 A1**
(43) Date of publication of application: **14.11.2018**
(21) Application number: 17750279.6
(22) Date of filing: 08.02.2017
(51) Int. Cl.: G06T 19/20, A61B 5/055, A61B 6/03, G06T 15/08

(54) **THREE-DIMENSIONAL IMAGE PROCESSING DEVICE, THREE-DIMENSIONAL IMAGE PROCESSING METHOD, AND THREE-DIMENSIONAL IMAGE PROCESSING PROGRAM**

(30) Priority: 09.02.2016 JP 2016022758
(71) Applicant: PHC Holdings Corporation, Tokyo 105-8433 (JP)
(72) Inventor: WATANABE, Katsuya, Ehime 791-0395 (JP); TAKEMURA, Tomoaki, Ehime 791-0395 (JP)
(74) Representative: Vigand, Philippe
(86) International application number: PCT/JP2017/004559
(87) International publication number: WO 2017/138558

(57) **Abstract**

Provided is a three-dimensional image processing device, comprising: an acquisition unit which acquires three-dimensional image data and metadata which is included in the three-dimensional image data; a separation unit which separates the three-dimensional image data which has been acquired with the acquisition unit into the three-dimensional image data and the metadata; a compositing unit which composites information, which is represented by the metadata which has been separated with the separation unit, with the three-dimensional image data which has been separated with the separation unit; and a transform unit which transforms the three-dimensional image data with which the information which is represented by the metadata has been composited in the compositing unit to three-dimensional shape model data which has a prescribed file format and which is of an object which is represented by the three-dimensional image data.

## Description

### Technical Field

The present invention relates to a three-dimensional image processing apparatus, a three-dimensional image processing method, and a three-dimensional image processing program for generating three-dimensional shape model data from three-dimensional image data.

### Background Art

As a conventional three-dimensional image processing apparatus of this type, for example, a stereoscopic model data generation apparatus disclosed in PTL 1 is known. In this stereoscopic model data generation apparatus, a liver region extraction section and a structure extraction section extract a structure such as a liver region, hepatic artery, or hepatic vein from three-dimensional image data, and a surface data generation section generates surface data of the liver region and the structure. A pattern imparting section imparts an uneven pattern to at least one of the surfaces of the liver region and the structure, and a data generation section synthesizes the surface data of the liver region and the structure to which the uneven pattern has been imparted to generate stereoscopic model data. A stereoscopic model creation apparatus creates a stereoscopic model of the liver on the basis of the stereoscopic model data.

As a conventional modeled object generated on the basis of three-dimensional shape model data, for example, a three-dimensional stereoscopic model disclosed in PTL 2 is known. This three-dimensional stereoscopic model is formed of a soft material that represents an external structure of a stereoscopic object, and an external color and an internal structure and an internal color that cannot be observed from the outside, and the internal structure and the internal color can be observed by cutting and opening the soft material.

### Citation List

### Patent Literature

PTL 1
   Japanese Patent No. 5814853
PTL 2
   Japanese Patent No. 3746779

### Summary of Invention

### Technical Problem

Meanwhile, metadata is added to three-dimensional image data in some cases. However, metadata has been lost in the process of creating three-dimensional shape model data from three-dimensional image data in a conventional three-dimensional image processing apparatus. Therefore, in order for a user to confirm a content of the metadata after creation of the three-dimensional shape model data, troublesome work such as checking by using the three-dimensional image data again is necessary.

Accordingly, an object of the present invention is to provide a three-dimensional image processing apparatus, a three-dimensional image processing method, and a three-dimensional image processing program capable of generating more convenient three-dimensional shape model data.

### Solution to Problem

A first aspect of the present invention is directed to a three-dimensional image processing apparatus including: an acquisition section that acquires three-dimensional image data including a three-dimensional image and metadata; a separation section that separates the three-dimensional image data acquired by the acquisition section into the three-dimensional image and the metadata; a synthesis section that synthesizes information represented by the metadata separated by the separation section with the three-dimensional image separated by the separation section; and a conversion section that converts the three-dimensional image synthesized with the information represented by the metadata in the synthesis section into three-dimensional shape model data of a predetermined file format and of an object represented by the three-dimensional image.

A second aspect of the present invention is directed to a three-dimensional image processing method including: an acquisition step of acquiring three-dimensional image data including a three-dimensional image and metadata; a separation step of separating the three-dimensional image data acquired in the acquisition step into the three-dimensional image and the metadata; a synthesis step of synthesizing information represented by the metadata separated by the separation step with the three-dimensional image separated by the separation step; and a conversion step of converting the three-dimensional image synthesized with the information represented by the metadata in the synthesis step into three-dimensional shape model data of a predetermined file format and of an object represented by the three-dimensional image.

A third aspect of the present invention is directed to a three-dimensional image processing program that causes a computer to perform: an acquisition step of acquiring three-dimensional image data including a three-dimensional image and metadata; a separation step of separating the three-dimensional image data acquired in the acquisition step into the three-dimensional image and the metadata; a synthesis step of synthesizing information represented by the metadata separated in the separation step with the three-dimensional image separated in the separation step; and a conversion step of converting the three-dimensional image synthesized with the information represented by the metadata in the synthesis step into three-dimensional shape model data of a predetermined file format and of an object represented by the three-dimensional image.

### Advantageous Effects of Invention

According to each embodiment described above, a three-dimensional image processing apparatus, a three-dimensional image processing method, and a three-dimensional image processing program capable of creating more convenient three-dimensional shape model data can be provided.

### Brief Description of Drawings

FIG. 1 is a diagram showing a hardware configuration and its peripheral configuration of a three-dimensional image processing apparatus according to Embodiments 1 and 2;
FIG. 2 is a diagram illustrating a data format of input three-dimensional image data;
FIG. 3 is a diagram showing functional blocks of a control section of FIG. 1;
FIG. 4A is a flow diagram showing a first half of a processing procedure of the three-dimensional image processing apparatus of FIG. 1;
FIG. 4B is a flow diagram showing a subsequent processing procedure of FIG. 4A;
FIG. 5 is a diagram showing each vertex coordinate of a polygon mesh composing three-dimensional shape model data and a normal vector of the polygon mesh;
FIG. 6 is a diagram showing a description example of three-dimensional shape model data in a binary format;
FIG. 7 is a diagram in which information (for example, characters) represented by metadata is divided into polygon meshes;
FIG. 8 is a diagram obtained by dividing an object (for example, a liver) represented by three-dimensional image data into polygon meshes;
FIG. 9 is a diagram exemplifying a three-dimensional modeled object output by a 3D modeling apparatus of FIG. 1;
FIG. 10 is a diagram exemplifying a three-dimensional modeled object related to a modified example of Embodiment 1;
FIG. 11 is a diagram showing functional blocks of a control section according to Embodiment 2;
FIG. 12 is a diagram exemplifying a three-dimensional modeled object according to Embodiment 2; and
FIG. 13 is a diagram showing a hardware configuration and its peripheral configuration of a three-dimensional image processing apparatus according to Embodiment 3.

### Description of Embodiments

### <<1. Embodiment 1>>

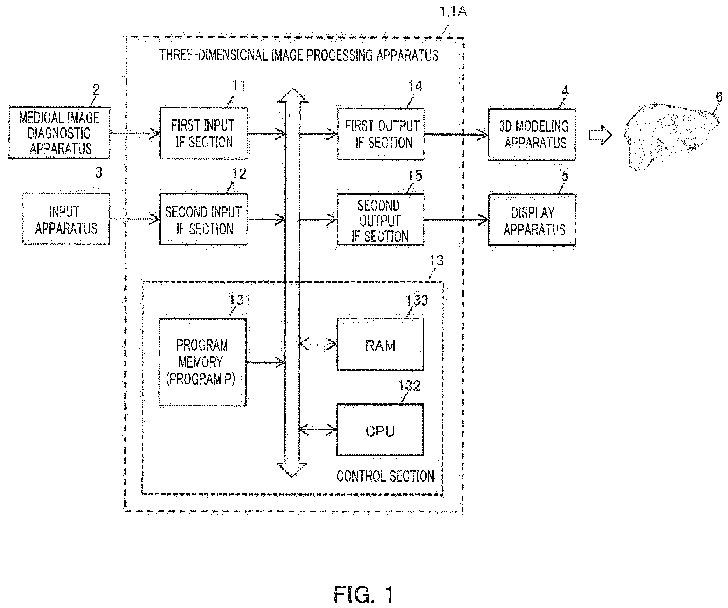

Three-dimensional image processing apparatus 1 according to Embodiment 1 of the present invention will be described in detail below with reference to FIGS. 1 to 10.

### << 1-1. Configuration of Three-Dimensional Image Processing Apparatus 1 >>

As shown in FIG. 1, three-dimensional image processing apparatus 1 includes first input IF section 11, second input IF section 12, control section 13, first output IF section 14, and second output IF section 15. The IF means an interface.

For example, medical image diagnostic apparatus 2 such as a computed tomography (CT) apparatus or a magnetic resonance imaging (MRI) apparatus can be connected to first input IF section 11. First input IF section 11 receives a three-dimensional image output from medical image diagnostic apparatus 2 under the control of control section 13 and stores the three-dimensional image in RAM 133.

Here, an example of the three-dimensional image will be described in detail. The three-dimensional image represents an image with a sense of depth, more specifically, a group of values (that is, volume data) assigned to each position on a three-dimensional space. This three-dimensional image also includes a collection of images (that is, two-dimensional tomographic images) that are obtained by medical image diagnostic apparatus 2 and are two-dimensional images stacked in a predetermined direction. In the present description, metadata is also added to the three-dimensional image. Although the metadata is not the three-dimensional image itself, but is additional information related to the three-dimensional image. Hereinafter, in the present description, data including a three-dimensional image and metadata added thereto are referred to as three-dimensional image data.

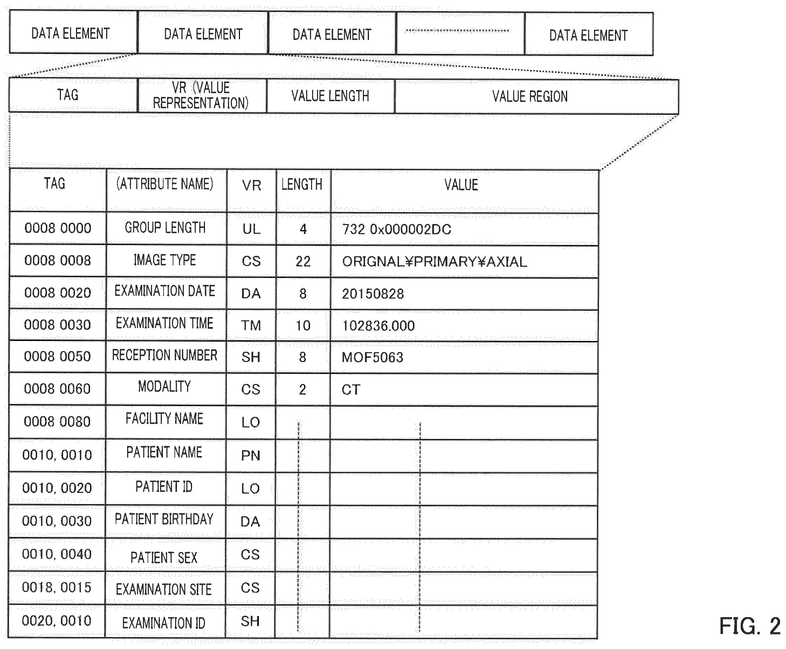

The three-dimensional image data has, for example, a DICOM format. The DICOM stands for digital imaging and communication in medicine and includes a standard defining the format of a medical image photographed by medical image diagnostic apparatus 2. As shown in FIG. 2, the DICOM format three-dimensional image data is a collection of data elements indicated by tags. Examples of metadata expressed by the tags include various data such as patient ID information or a patient name as information related to a patient, or an examination date as information related to an examination on the patient. In the DICOM, pixel values of three-dimensional images are also expressed using tags.

Reference is made to FIG. 1 again. Input apparatus 3 such as a keyboard or a mouse can be connected to second input IF section 12. Second input IF section 12 receives output data of input apparatus 3 under the control of control section 13 and transfers the output data to CPU 132.

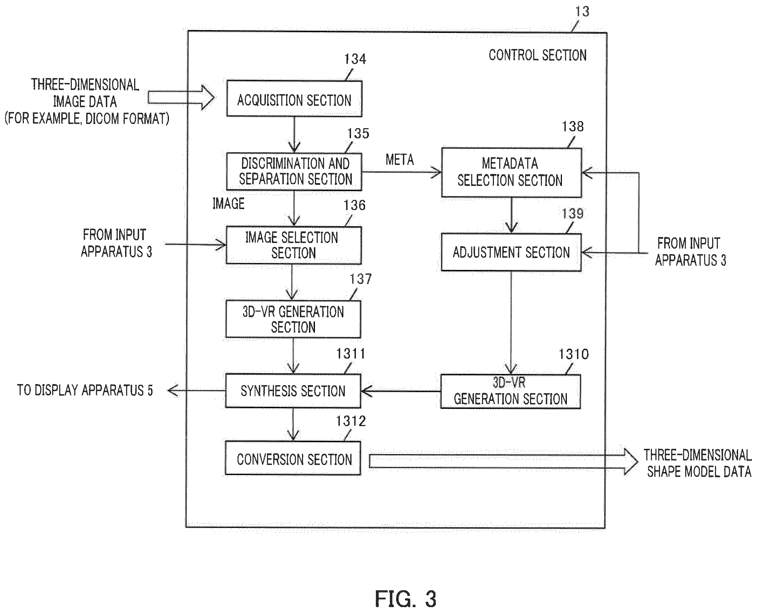

Control section 13 includes at least program memory 131, CPU 132, and RAM 133. Program memory 131 is, for example, a nonvolatile memory and stores three-dimensional image processing program P. CPU 132 executes program P while using RAM 133 as a work region, whereby, as shown in FIG. 3, operation is performed as each function block of acquisition section 134, discrimination and separation section 135, image selection section 136, 3D-VR generation section 137, metadata selection section 138, adjustment section 139, 3D-VR generation section 1310, synthesis section 1311, and conversion section 1312. The processing of each of function blocks 134 to 1312 will be described later.

Reference is made to FIG. 1 again. 3D modeling apparatus (so-called 3D printer) 4 can be connected to first output IF section 14. First output IF section 14 transfers three-dimensional shape model data generated by control section 13 to 3D modeling apparatus 4. 3D modeling apparatus 4 creates three-dimensional modeled object 6 on the basis of the received three-dimensional shape model data.

Display apparatus 5 such as a 2D or 3D high resolution display can be connected to second output IF section 15. Second output IF section 15 transfers various display data generated by control section 13 to display apparatus 5. Display apparatus 5 performs screen display on the basis of the received display data.

### <<1-2. Processing of Three-Dimensional Image Processing Apparatus 1>>

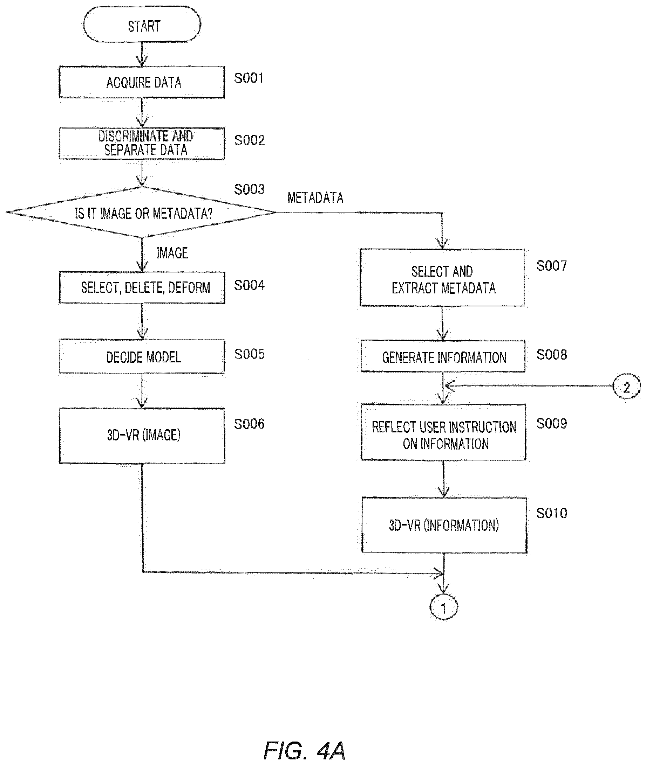

Three-dimensional image data output from medical image diagnostic apparatus 2 and having, for example, the DICOM format is input to first input IF section 11. In this description, it is assumed that the three-dimensional image data includes a two-dimensional tomographic image as an example of the three-dimensional image. Although the two-dimensional tomographic image represents a predetermined object, in the present description, it is assumed that the predetermined object is a predetermined human body site including at least the liver. In three-dimensional image processing apparatus 1, CPU 132 first functions as acquisition section 134 and controls input three-dimensional image data to first input IF section 11 to be transferred to RAM 133, whereby the three-dimensional data to be processed is acquired (step S001 in FIG. 4A).

Next, CPU 132 functions as discrimination and separation section 135, and when determining that the three-dimensional image data stored in RAM 133 conforms to the DICOM standard, on the basis of a value of each tag included in the three-dimensional image data or the like, separates a data element including the tag into a three-dimensional image (two-dimensional tomographic image in the present description) and metadata (step S002). As a result, RAM 133 separately stores the two-dimensional tomographic image photographed by medical image diagnostic apparatus 2 and the metadata related to the patient or examination, for example.

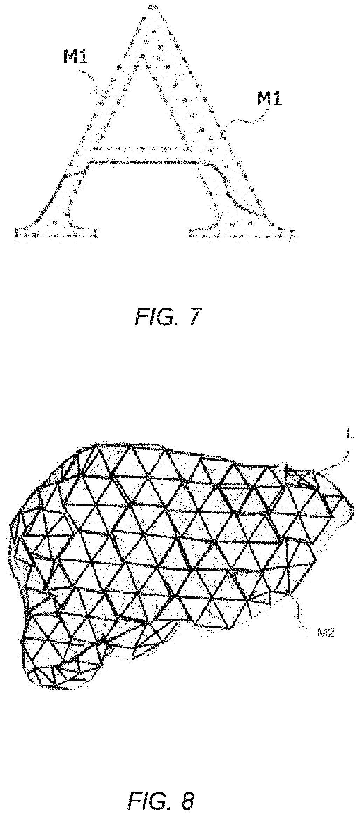

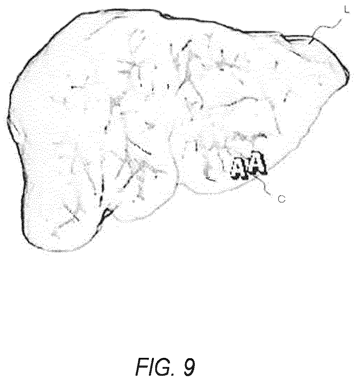

For the three-dimensional image (two-dimensional tomographic image) image data (image in step S003), CPU 132 processes as follows. By operating input apparatus 3, the user operates input apparatus 3 to transmit various instructions for extracting or deforming a necessary portion (that is, a human body site) from the two-dimensional tomographic image stored in RAM 133, or deleting an unnecessary portion to three-dimensional image processing apparatus 1. In this description, it is assumed that the user selects a two-dimensional tomographic image of the liver that is a human body site, and deletes other portions. In response to the operation of input apparatus 3, in three-dimensional image processing apparatus 1, CPU 132 functions as image selection section 136, processes the two-dimensional tomographic image as instructed from input apparatus 3 (step S004), and decides the portion to be a three-dimensional shape model (step S005). Thereafter, CPU 132 functions as 3D-VR generation section 137, performs 3D-VR (3D volume rendering) on the two-dimensional tomographic image decided in step S005 to generate a 3D-VR image (step S006). Here, the 3D-VR image represents a display image on display apparatus 5, and object L (see FIG. 8 and FIG. 9) obtained by integrating and projecting density values and color information of pixels along a predetermined viewing direction. In this description, object L is the liver. Since 3D-VR is well-known, detailed explanation thereof will be omitted.

In contrast to the above, CPU 132 processes metadata (metadata in step S003) as follows. That is, CPU 132 functions as metadata selection section 138 and selects and extracts necessary metadata from the metadata stored in RAM 133 according to an instruction from input apparatus 3 (step S007).

Next, CPU 132 functions as adjustment section 139 and generates information (for example, a character string) represented by the metadata selected in step S007 (step S008). Thereafter, the user operates input apparatus 3 to instruct three-dimensional image processing apparatus 1 a language, size, font, layout, etc. of the character string generated in step S008. As instructed from input apparatus 3, CPU 132 reflects the instruction of the user in the character string generated in step S008 (step S009). Thereafter, CPU 132 functions as 3D-VR generation section 1310 to generate 3D-VR metadata by performing 3D-VR on the information generated in step S008 (step S010). As similar to the 3D-VR image, the 3D-VR metadata also is display image data on display apparatus 5, and represents an object (character string in the present description) obtained by integrating and projecting density values and color information of pixels along a predetermined viewing direction. Since the procedure of 3D-VR is well-known, detailed description thereof will be omitted.

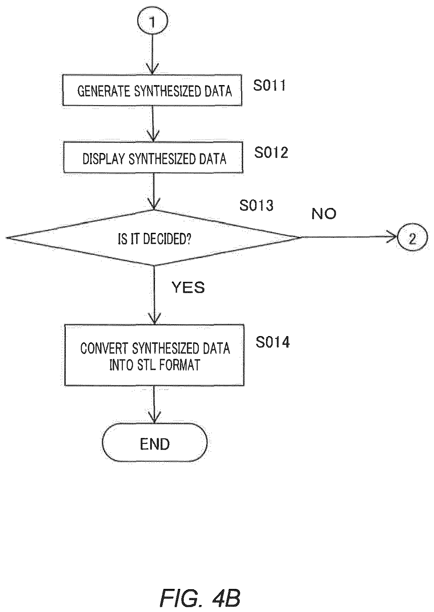

Upon completion of steps S006 and S010, CPU 132 functions as synthesis section 1311 to synthesis the 3D-VR metadata generated in S010 with the 3D-VR image generated in step S006 on RAM 133 to generate synthesized data (step S011 in FIG. 4B). This synthesized data represents the 3D-VR image obtained by synthesizing information represented by the 3D-VR metadata.

Next, CPU 132 transfers the synthesized data generated in step S011 to display apparatus 5 via second output IF section 15. In response to this, display apparatus 5 displays an image in which the information (character string in the present description) generated in step S008 has been synthesized with the portion (liver in the present description) decided in step S005 (step S012).

Next, CPU 132 determines whether the user has performed the decision operation with input apparatus 3 (step S013). The user refers to the display image in step S012 and starts modifying the character string for reasons such as poor visibility of the character string. In this case, CPU 132 determines that the user has not performed the decision operation, and reflects the instruction of the user to the information generated in step S008 as instructed from input apparatus 3 in step S009.

On the other hand, in step S013, when determining that the user has performed the decision operation by input apparatus 3, CPU 132 determines that there is no more modification, and converts the synthesized data generated in step S011 to three-dimensional shape model data of a standard triangulated language (STL) format (step S014). The STL format is generally called a stereolithography format.

The three-dimensional shape model data of the STL format generated by the above procedure is output to 3D modeling apparatus 4, for example, in order to formulate three-dimensional modeled object 6. Also, the configuration is not limited to this, and the three-dimensional shape model data may be stored in a portable storage medium or a remote server apparatus.

### <<1-3. Three-Dimensional Shape Model Data and Three-Dimensional Modeled Object 6>>

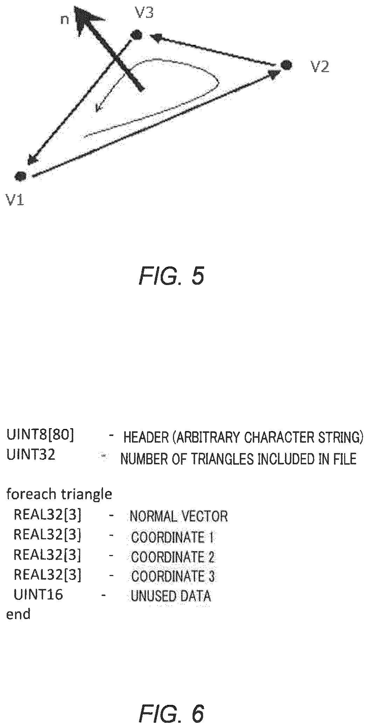

Here, the three-dimensional shape model data in the STL format will be described. This three-dimensional shape model data expresses an object with an aggregate of polygon meshes made of, for example, minute triangles. As shown in FIG. 5, each of such polygon meshes is defined by vertex coordinates V1, V2, V3 and triangle normal vector n.

FIG. 6 shows a description example of the three-dimensional shape model data in a binary format. For the STL format, the ASCII format is also prepared, but since a code amount is very large in the ASCII format, a binary format is frequently used when the three-dimensional shape model data is created in the STL format. In a case of the binary format, as shown in FIG. 6, the three-dimensional shape model data is started with an arbitrary character string of 80 bytes, and then the number of polygon meshes included in the three-dimensional shape model data is indicated by an integer of 4 bytes. Next, the normal vectors and the vertex coordinates for each polygon mesh continue by the number of the polygon meshes. There is no particular end code, and once the normal vectors and the vertex coordinates for the last triangle are described, the three-dimensional shape model data simply ends.

As a representative example of information represented by metadata, a method of converting "A" of the alphabet to three-dimensional shape model data is well known from before. In the case of creating three-dimensional data from the two-dimensional tomographic image data of medical image diagnostic apparatus 2, as shown in FIG. 7, modeling with a triangular polygon mesh is standard.

First, when converting the alphabet "A" into the three-dimensional shape model data, the alphabet "A" is divided into polygon meshes of a predetermined size. At this time, depending on the size of the smallest polygon mesh, when the total number of polygon meshes is large, the three-dimensional shape of the character string can be represented more finely, and conversely when the total number of polygon meshes is small, only coarse three-dimensional shapes can be expressed. In the present description, it is assumed that an alphabet "A" is formed with about 100 polygon meshes. In FIG. 7, for convenience sake, a reference numeral M1 is added to two polygon meshes.

FIG. 8 is a diagram obtained by dividing object (liver in this description) L represented by the three-dimensional image into polygon meshes. In order to naturally synthesize the character string with the liver when object L is reconstructed with the polygon mesh, it is preferable that the size of polygon mesh M2 is automatically set so that 1 to N character strings are included in polygon mesh M2 composing object L. Alternatively, the size of the polygon mesh composing object L may be automatically set so that one character is included in adjacent polygon meshes in the polygon mesh composing object L. In FIG. 8, for convenience sake, the reference numeral M2 is added to one polygon mesh.

FIG. 9 shows three-dimensional modeled object 6 output by 3D modeling apparatus 4 on the basis of the three-dimensional shape model data obtained by synthesizing information (character string) C with object L shown in FIG. 8.

### <<1-4. Effect of Three-Dimensional Image Processing Apparatus 1>>

As described above, according to three-dimensional image processing apparatus 1, discrimination and separation section 135 separates the three-dimensional image data into a three-dimensional image and metadata. 3D-VR generation section 137 performs 3D volume rendering on the three-dimensional image, and 3D-VR generation section 1310 performs 3D volume rendering on the separated metadata. Synthesis section 1311 synthesizes the 3D-VR metadata on the generated 3D-VR image to generate synthesized data. Conversion section 1312 creates three-dimensional shape model data in which information C is synthesized with object L on the basis of the synthesized data. Therefore, when three-dimensional modeled object 6 based on the three-dimensional shape model data is formed in 3D modeling apparatus 4, as shown in FIG. 9, information C (for example, a character string "AA") is formed on the surface of object L. As described above, in this three-dimensional image processing apparatus 1, the metadata added to the input three-dimensional image data is utilized without being discarded, so three-dimensional shape model data that is more convenient than before can be provided. Information C formed on three-dimensional modeled object 6 is much more beautiful than the information by handwriting and sealing, is easy to see, and does not disappear even when being cleaned and sterilized with chemicals or the like.

According to the present embodiment, in the case of medical use, three-dimensional modeled object 6 faithfully reproducing an affected portion of a patient, ID information or name of the patient as an example of information C represented by the metadata, the examination date, and the like can be associated with each other automatically. Therefore, it is not necessary for the user to perform troublesome work such as writing the ID information and name of the patient, or the like on the three-dimensional modeled object manually after completing the three-dimensional modeled object. In addition to the above, three-dimensional image processing apparatus 1 exerts an exceptional effect that patient authentication in a preoperative plan can be reliably performed. Therefore, according to the present embodiment, it is possible to provide three-dimensional image processing apparatus 1 capable of generating three-dimensional shape model data that is easier to use than before.

### <<1-5. Modification of Three-dimensional Image Processing Apparatus 1>>

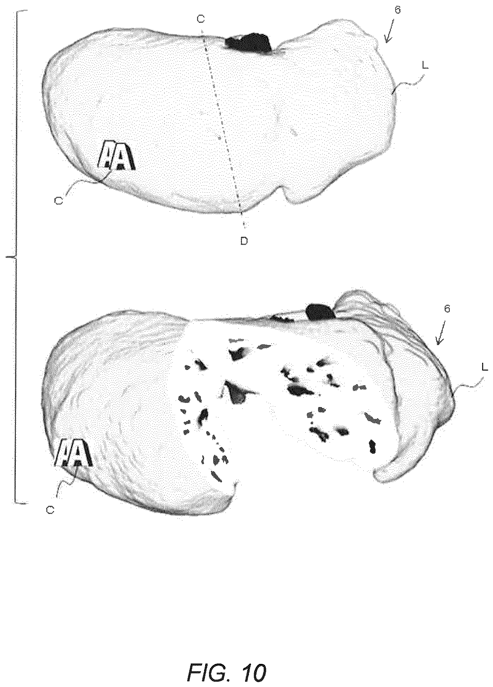

Three-dimensional modeled object 6 can also be used for training of resecting a tumor in a surgical operation, or the like. Here, in an upper part of FIG. 10, three-dimensional modeled object 6 before separation is shown together with a separation line C-D predetermined by a position of the tumor. In a lower part of FIG. 10, three-dimensional modeled object 6 after separation is shown, and a situation in which separation is proceeded, three-dimensional modeled object 6 is deformed, and a blood vessel or the like in object L is exposed. For such training, information C represented by the metadata is preferably synthesized around a polygon mesh having the creepage distance or the spatial distance from separation line C-D on the surface of three-dimensional modeled object 6.

When three-dimensional modeled object 6 is used in training applications, it is not necessary to synthesize ID information and the like of the patient with three-dimensional modeled object 6. However, when the sex or age the name of a doctor of the patient is synthesized instead of the ID information and the like of the patient, with three-dimensional modeled object 6 as another example of information C represented by the metadata, understanding of knowledge and technique on the case is deepened and beneficial.

According to the present embodiment, it is possible to automatically impart, for example, a serial No. or a manufacturing number necessary for mass production and additional production in three-dimensional image processing apparatus 1, or to add a stamp of a bar code of the manufacturing No. described later. Thus, quantity management by a serial No. in mass produced in lesson, or the like, visual confirmation of the manufacturing No. and additional ordering by visual confirmation of the manufacturing No. and reading the bar code stamp, and cost such as labor cost can also be lowered. This enables more effective utilization of a 3D model that is an output created by the 3D printer.

In order to automatically derive the synthesizing position as described above, the user operates input apparatus 3 and instructs separation line C-D. In step S009 of FIG. 4A, CPU 132 searches for a polygon mesh having the longest creeping distance or the like with respect to separation line C-D, and in step S011, synthesizes information C with the searched polygon mesh.

In the above description, it is described that information C is synthesized with the polygon mesh furthest from separation line C-D, but the configuration is not limited to this. Since a polygon mesh having the largest area or one or a plurality of polygon meshes having a small amount of deformation after separation also has distance from separation line C-D, information C may be synthesized with these polygon meshes.

In the above description, information C is synthesized with the polygon mesh furthest from separation line C-D. However, it is sufficient that the synthesis position of information C is determined with reference to a predetermined target position other than separation line C-D.

The synthesis position of information C derived by the above processing can be utilized as an initial position. More specifically, in step S011 in FIG. 4B, in brief, synthesized data in which information C is synthesized with the initial position on the image of the object surface is generated. Thereafter, the synthesis position of information C is corrected by the user operating input apparatus 3.

### <<2. Embodiment 2>>

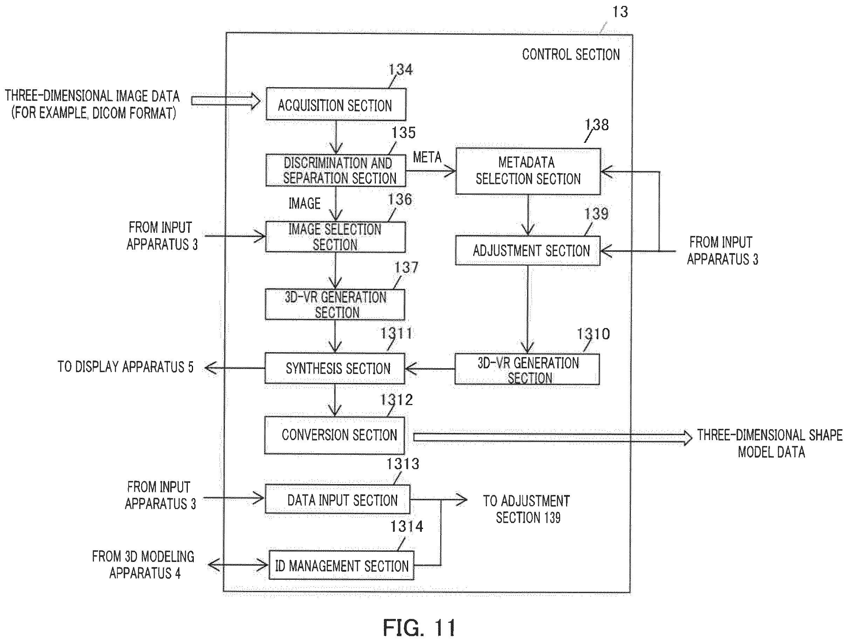

Next, three-dimensional image processing apparatus 1A according to Embodiment 2 of the present invention will be described in detail with reference to FIGS. 11 to 12.

### <<2-1. Configuration and Processing of Three-Dimensional Image Processing Apparatus 1A>>

Since a hardware configuration of three-dimensional image processing apparatus 1A is similar to that of three-dimensional image processing apparatus 1, FIG. 1 is cited.

CPU 132 executes program P stored in program memory 131 while using RAM 133 as a work region, whereby, as shown in FIG. 11, CPU 132 operates as data input section 1313 and ID management section 1314 in addition to function blocks 134 to 1312 described above.

In data input section 1313, data that is other than the metadata added to the three-dimensional image and is to be synthesized with object L is input.

As input data to data input section 1313, first, an enlargement ratio (0 < enlargement ratio) is exemplified. The cost of producing three-dimensional modeled object 6 by 3D modeling apparatus 4 is governed by the size of three-dimensional modeled object 6. Therefore, three-dimensional modeled object 6 smaller than the actual size may be produced unless three-dimensional modeled object 6 is not necessarily to be the actual size, such as for education for anatomy or the like, endoscopic surgery training, preoperative plan, or the like. However, the size with respect to the actual size (that is, the enlargement ratio) is important information. When this enlargement ratio is synthesized with three-dimensional modeled object 6, the user can recognize the enlargement ratio instantaneously and usability is high. Note that the enlargement ratio is typically input by operating input apparatus 3 by the user and passed to adjustment section 139 via data input section 1313.

As another input data, the name of the site and the name of the operation (procedure) are exemplified. In the DICOM tag, the site name represented by the two-dimensional tomographic image data can be described. However, in the present embodiment, since image selection section 136 can select a portion to be the three-dimensional shape model, the site name of object L represented by the three-dimensional shape model data is not always described with a tag of DICOM. For example, at the time of being transferred from medical image diagnostic apparatus 2 to this three-dimensional image processing apparatus 1A, the two-dimensional tomographic image represents both of the right lung and the left lung, and the site name in the tag of DICOM is described as "lungs". However, when the patient is suffering from emphysema in the left lung, three-dimensional shape model data is generated only for the left lung. In such a case, the user inputs the site name "left lung" just before the conversion in conversion section 1312 by operating input apparatus 3 or the like. The site name that has been input is passed to adjustment section 139 via data input section 1313.

Another input data is a name of creator, a creation date, or creation software name of three-dimensional modeled object 6, and there is naturally a difference in the quality of three-dimensional modeled object 6 depending on 3D modeling apparatus 4 and a material used for molding. When a 3D-VR image is generated from a two-dimensional tomographic image, a difference is generated in image quality or the like due to the performance of the CPU or the like. Therefore, even when the same two-dimensional tomographic image is used, there is a possibility that three-dimensional modeled objects 6 of the same quality may not be generated due to various factors. Accordingly, it can be considered from the standpoint of a doctor and a patient, that displaying such factors on three-dimensional modeled object 6 is desirable. The creator, creation date or creation software name can be input by the user operating input apparatus 3 in the same manner as the above-mentioned enlargement ratio. However, the configuration is not limited to this. User registration information and software license information held in three-dimensional image processing apparatus 1A may be transferred to data input section 1313 of control section 13 before conversion section 1312 converts the information into the STL format. As a result, it is possible to eliminate troublesome manual input by the user.

Another input data is information on 3D modeling apparatus 4. This information includes a manufacturer, an apparatus name, an apparatus number, or the like of 3D modeling apparatus 4. In order to acquire the information of 3D modeling apparatus 4, ID management section 1314 transmits a command to 3D modeling apparatus 4, acquires information from 3D modeling apparatus 4, and passes the information to adjustment section 139.

Adjustment section 139 treats each piece of external data received via data input section 1313 and ID management section 1314 in the same manner as the metadata. That is, adjustment section 139 generates information represented by the received external data in the same manner as the above-described step S008.

### <<2-2. Effect of Three-Dimensional Image Processing Apparatus 1A>>

According to Embodiment 2, it is possible to provide three-dimensional image processing apparatus 1A capable of generating three-dimensional shape model data that is easier to use, since various kinds of information can be displayed on three-dimensional modeled object 6.

### <<2-3. Modification of Three-Dimensional Image Processing Apparatus 1A>>

According to Embodiment 2, it is assumed that the information to be displayed on three-dimensional modeled object 6 increases. In this case, a one-dimensional code (so-called bar code) or a two-dimensional code (for example, a two-dimensional code standardized by JISX0510) generated from these pieces of information may be displayed on three-dimensional modeled object 6.

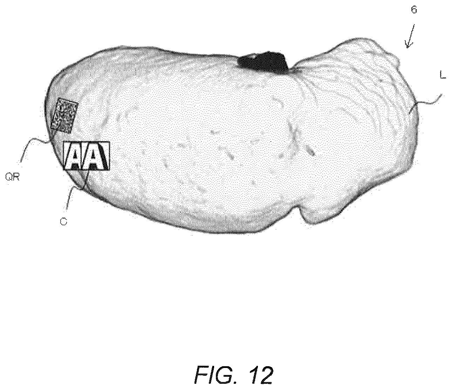

In some cases, it is preferable for a person to understand the meaning of the information displayed on three-dimensional modeled object 6, and there are cases where it is not so. Under such circumstances, as shown in FIG. 12, it is preferable that information (character) C is used for information that a person may understand, and other information is displayed on three-dimensional modeled object 6 using the two-dimensional code QR.

### <<3. Embodiment 3>>

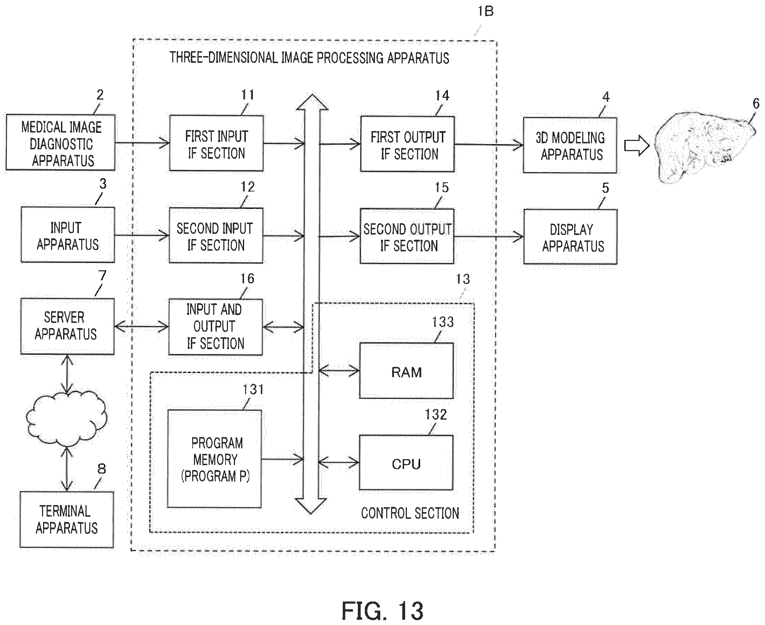

Next, three-dimensional image processing apparatus 1B according to Embodiment 3 of the present invention will be described in detail with reference to FIG. 13.

### <<3-1. Configuration and Processing of Three-Dimensional Image Processing Apparatus 1B>>

As shown in FIG. 13, three-dimensional image processing apparatus 1B is different from three-dimensional image processing apparatus 1A in that three-dimensional image processing apparatus 1B further includes an input and output IF section 16 that transmits and receives data to and from remote server apparatus 7. There is no more difference between two three-dimensional image processing apparatuses 1A, 1B. Therefore, in FIG. 13, those corresponding to the configurations shown in FIG. 1 are denoted by the same reference numerals, and description thereof will be omitted.

The input and output IF section 16 stores in remote server apparatus 7 the three-dimensional shape model data generated by control section 13 by the method described in Embodiment 1 and Embodiment 2.

Server apparatus 7 is, for example, managed by a dealer who sells the three-dimensional shape model data stored therein, and can be accessed by various terminal apparatus 8 such as a personal computer. For example, suppose that a doctor who participated in a case study meeting want to use three-dimensional modeled object 6 as shown in FIG. 12 in his own hospital after using three-dimensional modeled object 6 in the case study meeting. Here, it is assumed that addresses (that is, locators) on the network of server apparatus 7 are described in the two-dimensional code QR of three-dimensional modeled object 6. Under this assumption, the doctor can operates terminal apparatus 8, obtains the address of server apparatus 7, access server apparatus 7 to place an order for three-dimensional modeled object 6.

### <<3-2. Effect of Three-Dimensional Image Processing Apparatus 1B>>

As described above, according to three-dimensional image processing apparatus 1B, since the three-dimensional shape model data generated by three-dimensional image processing apparatus 1B itself can be stored in remote server apparatus 7, the three-dimensional shape model data can be used for a wider application.

### <<3-3. Appendix of Three-Dimensional Image Processing Apparatus 1B>>

When server apparatus 7 is also provided with three-dimensional image processing apparatus 1B, a serial number corresponding to the number of orders may be displayed as information C on three-dimensional modeled object 6 in order to prevent counterfeit products.

This application claims priority based on Japanese Patent Application Laid-Open No. 2016-022758 filed on February 9, 2016 to the Japan Patent Office. The contents of Japanese Patent Application Laid-Open No. 2016-022758 are incorporated into this application by reference.

### Industrial Applicability

The three-dimensional image processing apparatus according to the present invention can create three-dimensional shape model data that is more convenient and is suitable for medical use and the like.

### Reference Signs List

1, 1A, 1B Three-dimensional image processing apparatus
134 Acquisition section
135 Discrimination and separation section
136 Image selection section
138 Metadata selection section
1311 Synthesis section
1312 Conversion section

## Claims

1. A three-dimensional image processing apparatus comprising:
an acquisition section that acquires three-dimensional image data including a three-dimensional image and metadata;
a separation section that separates the three-dimensional image data acquired by the acquisition section into the three-dimensional image and the metadata;
a synthesis section that synthesizes information represented by the metadata separated by the separation section with the three-dimensional image separated by the separation section; and
a conversion section that converts the three-dimensional image synthesized with the information represented by the metadata in the synthesis section into three-dimensional shape model data of a predetermined file format and of an object represented by the three-dimensional image.

2. The three-dimensional image processing apparatus according to claim 1, wherein the acquisition section acquires three-dimensional image data generated by a predetermined medical image diagnostic apparatus and including metadata.

3. The three-dimensional image processing apparatus according to claim 1 or 2, further comprising a metadata selection section that selects predetermined metadata from the metadata separated by the separation section, wherein
the synthesis section synthesizes information represented by the predetermined metadata selected by the metadata selection section with the three-dimensional image separated by the separation section.

4. The three-dimensional image processing apparatus according to any one of claims 1 to 3, further comprising an image selection section that selects a predetermined three-dimensional image from the three-dimensional image separated by the separation section, wherein
the synthesis section synthesizes information represented by the metadata separated by the separation section with the predetermined three-dimensional image selected by the image selection section.

5. The three-dimensional image processing apparatus according to claim 4, wherein the image selection section selects a three-dimensional image representing a predetermined human body site from the three-dimensional image separated by the separation section.

6. The three-dimensional image processing apparatus according to any one of claims 1 to 5, wherein the three-dimensional image and the metadata conform to digital imaging and communication in medicine (DICOM).

7. The three-dimensional image processing apparatus according to any one of claims 1 to 6, wherein the metadata represents information related to a patient or an examination.

8. The three-dimensional image processing apparatus according to any one of claims 1 to 7, wherein
the synthesis section synthesizes information represented by the metadata separated by the separation section with the three-dimensional image separated by the separation section such that the information represented by the metadata is arranged in a predetermined position in an image of an object surface represented by the three-dimensional image; and
the predetermined position is a position apart from a target position in the image of the object surface by a predetermined distance.

9. The three-dimensional image processing apparatus according to any one of claims 1 to 8, wherein the metadata represents character string information.

10. The three-dimensional image processing apparatus according to any one of claims 1 to 9, wherein the synthesis section further synthesizes information other than the information represented by the metadata separated by the separation section with the three-dimensional image separated by the separation section.

11. A three-dimensional image processing method comprising:
an acquisition step of acquiring three-dimensional image data including a three-dimensional image and metadata;
a separation step of separating the three-dimensional image data acquired in the acquisition step into the three-dimensional image and the metadata;
a synthesis step of synthesizing information represented by the metadata separated by the separation step with the three-dimensional image separated by the separation step; and
a conversion step of converting the three-dimensional image synthesized with the information represented by the metadata in the synthesis step into three-dimensional shape model data of a predetermined file format and of an object represented by the three-dimensional image.

12. A three-dimensional image processing program that causes a computer to perform:
an acquisition step of acquiring three-dimensional image data including a three-dimensional image and metadata;
a separation step of separating the three-dimensional image data acquired in the acquisition step into the three-dimensional image and the metadata;
a synthesis step of synthesizing information represented by the metadata separated in the separation step with the three-dimensional image separated in the separation step; and
a conversion step of converting the three-dimensional image synthesized with the information represented by the metadata in the synthesis step into three-dimensional shape model data of a predetermined file format and of an object represented by the three-dimensional image.
